# EUROPÄISCHE PATENTSCHRIFT

(11) **EP 1 393 761 B1**
(45) Veröffentlichungstag und Bekanntmachung des Hinweises auf die Patenterteilung: **03.12.2008**
(21) Anmeldenummer: 03017969.1
(22) Anmeldetag: 06.08.2003
(51) Int. Cl.: A61M 1/16

(54) **Bilanzkammer für eine Vorrichtung zum Fördern von Flüssigkeiten für eine medizinische Behandlungsvorrichtung**
Balance chamber for a device conveying fluids in a medical treatment decive
Chambre d'equilibrage pour un dispositif transportant des liquides à utiliser dans un dispositif de traitement médical

(30) Priorität: 28.08.2002 DE 10239598
(43) Veröffentlichungstag der Anmeldung: 03.03.2004
(73) Patentinhaber: Fresenius Medical Care Deutschland GmbH, 61352 Bad Homburg v.d.H. (DE)
(72) Erfinder: Brauer, Helge, 97469 Gochsheim (DE); Ehrenberger, Walter, 97447 Gerolzhofen (DE); Ender, Helmuth, 97475 Zeil (DE); Noack, Joachim, Dr., 97616 Bad Neustadt (DE)
(74) Vertreter: Laufhütte, Dieter

(56) Entgegenhaltungen:
- EP-A- 0 306 241
- DE-A- 3 016 720
- US-A- 4 267 040
- US-A- 4 366 061
- US-A- 4 431 425
- US-A- 5 580 460

## Beschreibung

Die Erfindung betrifft eine Bilanziervorrichtung für eine medizinische Behandlungsvorrichtung nach dem Oberbegriff des Anspruchs 1.

Zur Entfernung von harnpflichtigen Substanzen und zum Flüssigkeitsentzug werden beim akuten und chronischen Nierenversagen verschiedene Verfahren zur operativen Blutreinigung bzw. Blutbehandlung eingesetzt. Bei der Hämodialyse (HD) überwiegt der diffuse Stofftransport, während bei der Hämofiltration (HF) ein konvektiver Stofftransport über eine Membran vorliegt. Eine Kombination aus beiden Verfahren ist die Hämodiafiltration (HDF). Bei der Peritonealdialyse (PD) wird das Peritoneum als Kontaktmembran ausgenützt.

Wegen der großen Austauschmengen besteht bei den genannten Verfahren die Notwendigkeit einer exakten Bilanzierung der dem Patienten entzogenen Flüssigkeit und der dem Patienten zugeführten Flüssigkeit über die gesamte Behandlungszeit. Zum Stand der Technik gehören gravimetrische und volumetrische Bilanziervorrichtungen.

Eine Hämodiafiltrationsvorrichtung mit volumetrischer Bilanzierung ist beispielsweise aus der DE 26 34 238 A1 bekannt. Die Bilanziervorrichtung der bekannten Hämodiafiltrationsvorrichtung weist einen volumenstarren Hohlkörper auf, der durch eine bewegliche Trennwand in zwei Kammern unterteilt ist. Jede Kammer weist einen Einlaß und einen Auslaß auf, an denen Zuführ- und Abführleitungen für frische bzw. verbrauchte Dialysierflüssigkeit angeordnet sind, wobei in jeder Leitung ein Absperrorgan geschaltet ist. Darüber hinaus sind Pumpen für die Förderung der frischen und verbrauchten Dialysierflüssigkeit sowie eine Steuereinheit vorgesehen, die ein wechselseitiges Befüllen der beiden Kammern erlaubt. In der Zuflußleitung der ersten und zweiten Kammer ist jeweils eine Druckmeßeinrichtung angeordnet, die den Druck in der Leitung überwacht. Wenn eine Kammer gefüllt ist, erfolgt ein Druckanstieg, der die entsprechende Pumpe abschaltet.

Aus US 4,366,061 ist weiterhin eine Bilanziervorrichtung nach dem Oberbegriff von Anspruch 1 bekannt, bei welcher zwei Bilanzkammem mit jeweils einer ersten und einer zweiten Teilkammer vorgesehen sind, welche abwechselnd mit den Zuführ-und Abführleitungen für frische bzw. verbrauchte Dialysierflüssigkeit verbunden werden. Dabei werden für jeden Halbzyklus die Füllzeiten der soeben befüllten Teilkammern der beiden Bilanzkammern miteinander verglichen, um die Antriebsleistung der jeweiligen Antriebe entsprechend anzupassen.

Die US 4,431,425 A beschreibt eine Vorrichtung zur Lageerkennung der beweglichen Trennwand einer Bilanziervorrichtung mit einem optischen Detektor, der außerhalb der Bilanzierkammer angeordnet ist. Die bekannte Vorrichtung ist insofern nachteilig, als das Gehäuse der Bilanzierkammer aus transparentem Material bestehen muß.

Aus der DE 197 28 800 C1 ist eine Vorrichtung zum Fördern von Flüssigkeiten für eine medizinische Behandlungsvorrichtung bekannt, bei der in einer Bilanzkammer Druckimpulse pro Zeit aufgenommen werden. Damit wird die Frequenz von Bilanzkammerhüben pro Zeit festgestellt. Hier geht es um eine Überwachung der vollständigen Füllung und Entleerung der Bilanzkammer.

Aus der DE 28 38 414 C2 ist ebenfalls ein Bilanzkammersystem bekannt, gemäß dem sich mit Hilfe eines Leitfähigkeitsüberwachungssystems ein gegebenenfalls in der membranartigen Trennwand der entsprechenden Förderkammer bzw. Bilanzkammer auftretendes Leck detektiert werden kann. Ein solches Leck ist unerwünscht, da es zu einer Vermengung von frischer und benutzter Dialysierflüssigkeit führt, was insbesondere eine Veränderung der Behandlungseffektivität nach sich zieht. Zusätzlich ist der Füllzustand der Bilanzkammer schwierig zu detektieren, da der Druckanstieg am Ende des Umwälzzyklus nicht sehr deutlich ausgeprägt ist. Eine Leckerkennung über eine Leitfähigkeitsüberwachung kann allerdings nicht sehr genau arbeiten, da sich die Leitfähigkeit der vermischten Flüssigkeiten kaum voneinander unterscheiden. Insbesondere kleine Lecks können praktisch nicht detektiert werden.

Aufgabe der Erfindung ist es daher, eine gattungsgemäße Bilanziervorrichtung für eine medizinische Behandlungsvorrichtung derart weiter zu bilden, daß eventuell in der membranartigen beweglichen Trennwand auftretende Lecks sicher erkennbar sind.

Erfindungsgemäß wird diese Aufgabe durch eine Kombination der Merkmale des Anspruchs 1 gelöst. Demnach werden bei einer gattungsgemäßen Bilanziervorrichtung für eine medizinische Behandlungsvorrichtung mittels der Auswerteeinrichtung zunächst die Füllzeiten der Teilkammern festgestellt und anschließend werden jeweils die Füllzeiten der ersten Teilkammern der ersten und zweiten Bilanzkammer und/oder der zweiten Teilkammern der ersten und zweiten Bilanzkammern miteinander verglichen. Die Erfindung basiert auf der Erkenntnis, daß bei einem vorgegebenen Volumenstrom die Füllzeiten zum Auffüllen des Teilkammervolumens konstant sind. Sollte es nun zu einem Unterschied der Füllzeiten der parallel geschalteten ersten Teilkammern der Bilanzkammern bzw. der zweiten Teilkammern der Bilartzkammern kommen, ist dies ein Anzeichen für ein Leck in der beweglichen Trennwand.

Die Füllzeiten können beispielsweise in einem Speicher als Vergleichswerte für die jeweils aktuell gemessenen Füllzeiten abgelegt sein. Bei Abweichung der Füllzeiten um einen vorgegebenen Wert ΔT kann die Auswerteeinrichtung auf ein entsprechendes Leck in der Trennwand schließen.

Gemäß einer bevorzugten Ausführungsvariante der Erfindung ist in der Vorrichtung zusätzlich ein optischer und/oder akustischer Signalgenerator enthalten, über den ein Alarmsignal abgegeben werden kann, falls ein Leck in der beweglichen Trennwand festgestellt wurde.

Schließlich kann das Leckerkennungssignal erst dann ausgebbar sein, wenn das Überschreiten der vorgegebenen Zeitdifferenz nach vorgegebenen Kriterien mehrfach auftritt. Hierdurch können Fehlalarme sicher verhindert werden.

Mit der erfindungsgemäßen Vorrichtung findet nun ein Vergleich zwischen den Füllzeiten jeweils einer Teilkammer der ersten Bilanzkammer mit jeweils einer entsprechenden Teilkammer der zweiten Bilanzkammer statt. Dadurch werden Einflüsse durch Flußpumpen, Strömungsprofile oder dergleichen herauskompensiert. Das Ablegen von entsprechenden Kalibrierzeiten in einem Speicher der Auswerteeinrichtung ist daher in vorteilhafter Weise nicht notwendig.

Weitere Einzelheiten und Vorteile der Erfindung werden anhand eines in der Zeichnung dargestellten Ausführungsbeispiels näher erläutert. Es zeigt die einzige Figur

ein vereinfachtes Prinzipschema eines Ausführungsbeispiels der erfindungsgemäßen Vorrichtung.

In der Figur ist der Dialysierflüssigkeitsverlauf 100 einer erfindungsgemäßen Hämodialysevorrichtung mit volumetrischer Bilanzierung ebenfalls in stark vereinfachter Darstellung gezeigt. Die Bilanziervorrichtung besteht aus den Bilanzkammern 122 und 123 und den zugehörigen Ventilen 114 bis 121. Wesentlicher Bestandteil der Bilanziervorrichtung sind die beiden Kammern 122 und 123. Dem Prinzip nach bestehen diese aus volumenstarren Hohlkörpern mit je zwei Räumen, die durch ein bewegliches, dicht schließendes Element 124 bzw. 125 voneinander getrennt sind, so daß bei einer Vergrößerung des einen Raumes der andere Raum zwangsläufig um den gleichen Betrag verkleinert wird. In der schematischen Darstellung der Figur sind die Kammern 122 und 123 beispielsweise als Kugeln und die beweglichen Elemente 124 und 125 als Membran dargestellt. Wichtig für die tatsächliche Gestaltung der Kammern und der darin beweglichen Elemente im Hinblick auf die Dosierfunktion ist außerdem, daß die Verschiebung der beweglichen Elemente von einer Extremlage in die andere zu einer reproduzierbaren Volumenverschiebung führt. Bei der in der Figur angedeuteten Ausführung wird dies beispielsweise dadurch erreicht, daß die Membran 124 und 125 in ihren Extremlagen vollständig gegen die rechte oder linke Wand der jeweiligen Kammer anliegen, so daß bei der Bewegung von einer Extremlage in die andere eine Volumenverschiebung vom Betrage des gesamten Kammervolumens stattfindet.

Die den Bilanzkammern zugeordneten Ventile 114 bis 121 bilden zwei Gruppen, die wechselseitig betätigt werden. Wenn die Ventile der Gruppe A (115, 117, 118, 120) geöffnet sind, sind die Ventile der Gruppe B (114, 116, 119, 121) geschlossen, und umgekehrt. Die beiden Kammern arbeiten dadurch abwechselnd, wobei sie periodisch ihre Funktionen vertauschen. Während jeweils eine der beiden Kammern in den Kreislauf (102a, 103a) des Dialysators 104 eingefügt ist, wird die andere Kammer mit neuer Dialysierlösung über die Zulaufleitung 102 geladen und gleichzeitig die gebrauchte Dialysierlösung in die Abflußleitung 103 verdrängt.

Wenn die Ventile der Gruppe A (dunkel dargestellt) geöffnet und die Ventile der Gruppe B (hell dargestellt) geschlossen sind, wird die Kammer 122 mit frischer Dialysierlösung geladen, während die Kammer 123 zur Speisung des Dialysators dient. Der Ladevorgang der Kammer 122 ergibt sich dadurch, daß durch das geöffnete Ventil 118 frische Lösung unter Druck in den Raum 122a strömt, so daß die Membran 124 ausweicht und die jenseits der Membran in Raum 122b befindliche gebrauchte Dialysierlösung durch das geöffnete Ventil 115 in die Abschlußleitung 103 verdrängt wird. Wenn sich die Membran ganz an die rechte Kammerwand angelegt hat, ist dieser Ladevorgang beendet.

Aus der Kammer 123 wird währenddessen der Dialysator gespeist, indem die in Raum 123a befindliche frische Dialysierlösung durch das geöffnete Ventil 117 über die Leitung 102a zum Dialysator geleitet und als gebrauchte Dialysierlösung vom Dialysator über die Leitung 103a und das geöffnete Ventil 120 in den Raum 123b der gleichen Kammer zurückgeführt wird. Aufgrund der Volumenstarrheit der Bilanzkammer muß die zurückgeführte Flüssigkeitsmenge genau mit der dem Dialysator zugeführten Flüssigkeitsmenge übereinstimmen. Die Dialysierlösung fließt hierbei in einem quasi geschlossenen Kreislauf, weil Anfang und Ende über das verschiebbare Element in der Bilanzkammer miteinander verbunden sind. Eine Vermischung von frischer und gebrauchter Dialysierlösung findet aber nicht statt. Sobald sich die Membran in der Kammer 123 vollständig an die rechte Kammerwand angelegt hat, ist der Vorgang beendet. Um den Durchfluß durch den Dialysator 104 weiter aufrecht zu erhalten, werden nur die Ventile umgeschaltet, so daß die beiden Kammern 122 und 123 der Bilanziervorrichtung ihre Funktion vertauschen.

Wenn nun die Ventile der Gruppe A geschlossen und die Ventile der Gruppe B geöffnet sind, kann dem Dialysator durch das geöffnete Ventil 114 aus dem Raum 122a der Kammer 122 weiter frische Dialysierlösung zufließen, während die gleich große Menge gebrauchter Dialysierlösung aus dem Dialysator über das geöffnete Ventil 119 in den Raum 122b auf der anderen Seite der Membran zugeführt wird. Zu Beginn dieses Vorganges befindet sich der Raum 122a im maximalen und der Raum 122b im minimalen Füllzustand, da beim vorhergehenden Arbeitstakt der Raum 122a, wie beschrieben, vollständig mit frischer Dialysierlösung gefüllt wurde. Während der Dialysator aus der Kammer 122 gespeist wird, wird die Kammer 123, deren Raum 123b vom vorherigen Arbeitstakt vollständig mit gebrauchter Dialysierlösung gefüllt ist, mit frischer Dialysierlösung aufgeladen. Die frische Dialysierlösung fließt über das geöffnete Ventil 121 in den Raum 123a, und die im Raum 123b befindliche gebrauchte Dialysierlösung wird durch das geöffnete Ventil 116 in die Abflußleitung 103 verdrängt.

Die Umschaltung der Ventilgruppen muß jeweils dann erfolgen, wenn der Vorrat der Bilanzkammer, aus der im Augenblick der Dialysator 104 gespeist wird, erschöpft ist. Die Aufladung der anderen Bilanzierkammer soll zu diesem Zeitpunkt bereits abgeschlossen sein, was durch eine entsprechend hohe Ladegeschwindigkeit ohne weiteres erreichbar ist. Das Signal für die Umschaltung der Ventile kann auf verschiedene Weise gewonnen werden. Da der Dialysierlösungsfluß im Dialysatorkreis aufhört, sobald die Membran in der den Dialysator 104 speisenden Bilanzkammer ihre Extremstellung erreicht hat, könnte z. B. ein Durchflußmesser mit der Einrichtung zur Signalauslösung bei Unterschreitung eines Minimalflusses für diesen Zweck benutzt werden. Eine andere Möglichkeit besteht darin, eine durch das Erreichen der Endlage bedingte Druckveränderung zur Auslösung der Umschaltfunktion auszunutzen. Eine wiederum andere Möglichkeit besteht darin, die erhöhte Leistungsaufnahme der Ladepumpe am Ende eines Fülltaktes zur Endlagenerkennung zu nutzen.

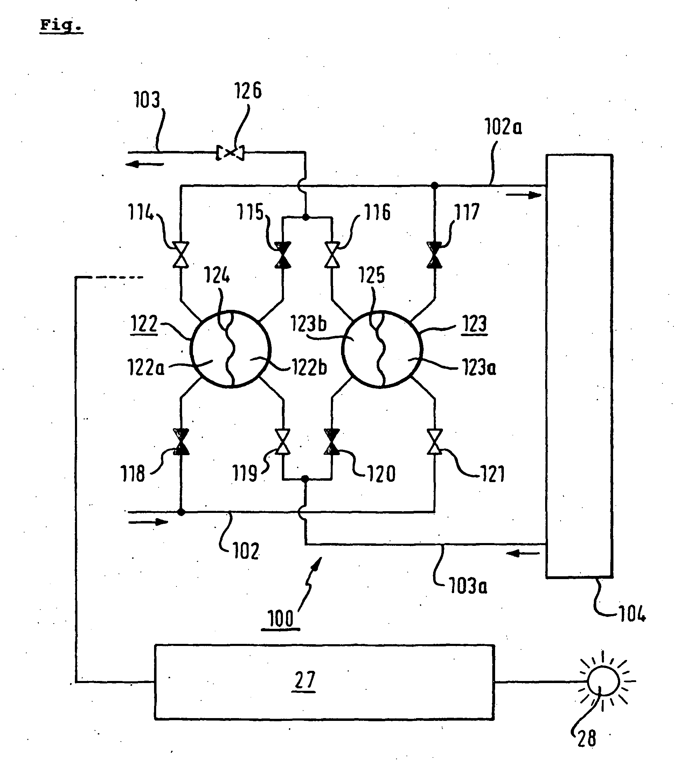

Zur Erkennung einer unvollständigen Füllung bzw. Entleerung der jeweiligen Teilkammern 122a bzw. 122b und 123a bzw. 123b der Bilanzkammern 122 und 123 ist eine Überwachungseinrichtung 27 vorgesehen, die eine außerhalb der Bilanzierkammer angeordnete Druckmeßeinrichtung sowie eine Auswerte- und Recheneinheit 27 umfaßt. In der Figur 3 ist die Recheneinheit aus Vereinfachungsgründen nur sehr schematisiert dargestellt, d. h. ohne die Anordnung der einzelnen Druckmeßeinrichtungen sowie der jeweiligen Verbindungsleitungen. Die Druckmeßeinrichtungen müssen jeweils mindestens an entweder die ersten Teilkammern 122a und 123a oder die zweiten Teilkammern 122b und 123b angeschlossen sein, was auch über die Zuführ- bzw. Abführleitungen erfolgen kann.

Mittels der Auswerteeinheit 27 werden nun die Füllzeiten der Teilkammern 122a und 123a bzw. 122b und 123b festgestellt. Bei Abweichung der Füllzeiten für die jeweils zu befüllende Teilkammer 122a bzw. 123a einerseits und 122b bzw. 123b andererseits um ein Zeitintervall ΔT wird ein Leckerkennungssignal ausgegeben, mittels dem ein optischer Signalgenerator 28 ansteuerbar ist.

## Patentansprüche

1. Bilanziervorrichtung für eine medizinische Behandlungsvorrichtung mit zwei Bilanzkammern (122, 123) gleichen Volumens, die je von einer beweglichen Trennwand (124, 125) in eine erste und eine zweite Teilkammer (122a, b; 123a, b) unterteilt sind, wobei die Teilkammern jeweils einen Zufluß und einen Abfluß aufweisen und wobei die Zuflüsse der ersten Teilkammern (122a, 123a) eine gemeinsame erste Zuflußleitung (102a) aufweisen und die Zuflüsse der zweiten Teilkammern (122b, 123b) eine gemeinsame zweite Zuflußleitung (103a) aufweisen, und mit einer Auswerteeinrichtung (27),
**dadurch gekennzeichnet,**
**daß** die Auswerteeinrichtung (27) so ausgestaltet ist, dass sie im Gebrauch zunächst die Füllzeiten der ersten Teilkammern (122a, 123a) feststellt und anschließend die Füllzeiten der ersten Teilkammern (122a, 123a) der ersten und zweiten Bilanzkammer (122 bzw. 123) miteinander vergleicht zur Bestimmung eines Unterschieds der Füllzeiten der ersten Teilkammern als Anzeichen für ein Leck in der beweglichen Trennwand.

2. Bilanziervorrichtung nach Anspruch 1, **dadurch gekennzeichel, daß** die Auswerteeinrichtung (27) so ausgestaltet ist, dass sie im Gebrauch zuterm zunächst die Füllzeiten der zweiten Teilkammern (122b, 123b) feststellt und anschließend die Füllzeiten der zweiten Teilkammern (122b, 123b) der ersten und zweiten Bilanzkammer (122 bzw. 123) miteinander vergleicht.

3. Bilanziervorrichtung nach Anspruch 1 oder 2, **dadurch gekennzeichnet, daß** die Auswerteeinrichtung so ausgestaltet ist, dass sie im Gebrauch bei Überschreiten einer vorgegebenen Zeitdifferenz (ΔT) ein Leckerkennungssignal ausgist.

4. Bilanziervorrichtung nach Anspruch 3, **dadurch gekennzeichnet, daß** sie einen optischen und/ oder akustischen Signalgenerator (28) aufweist.

5. Bilanziervorrichtung nach Anspruch 2, **dadurch gekennzeichnet, dass** die Auswerteeinrichtung so ausgestaltet ist, dass sie im Gebrauch das Leckerkennungsslgnal erst dann ausgist, wenn das Überschreiten der vorgegebenen Zeitdifferenz (ΔT) nach vorgegebenen Kriterien mehrfach auftritt.

## Claims

1. A balancing device for a medical treatment apparatus comprising two balancing chambers (122, 123) of equal volume, which are respectively subdivided by a movable partition (124, 125) into a first and a second chamber portion (122a, b; 123a, b), wherein the chamber portions respectively have a supply means and a discharge means, and wherein the supply means of the first chamber portions (122a, 123a) have a common first supply line (102a) and the supply means of the second chamber portions (122b, 123b) have a common second supply line (103a), and comprising an evaluation device (27),
**characterised in that**
the evaluation device (27) is so adapted that in use it firstly detects the filling times of the first chamber portions (122a, 123a) and then compares together the filling times of the first chamber portions (122a, 123a) of the first and second balancing chambers (122 and 123 respectively) to determine a difference in the filling times of the first chamber portions as an indication of a leak in the movable partition.

2. A balancing device according to claim 1 **characterised in that** the evaluation device (27) is so adapted that in use it also firstly detects the filling times of the second chamber portions (122b, 123b) and then compares together the filling times of the second chamber portions (122b, 123b) of the first and second balancing chambers (122 and 123 respectively).

3. A balancing device according to claim 1 or claim 2 **characterised in that** the evaluation device is so adapted that in use it outputs a leak detection signal when a predetermined time difference (ΔT) is exceeded.

4. A balancing device according to claim 3 **characterised in that** it has an optical and/or acoustic signal generator (28).

5. A balancing device according to claim 3 **characterised in that** the evaluation device is so adapted that in use it outputs the leak detection signal only when the predetermined time difference (ΔT) in accordance with predetermined criteria is exceeded a plurality of times.

## Revendications

1. Dispositif d'équilibrage pour un dispositif de traitement médical avec deux chambres d'équilibrage (122, 123) d'un même volume, qui sont séparées chacune par une paroi de séparation mobile (124, 125) en une première et une deuxième chambre partielle (122a, b; 123a, b), où les chambres partielles présentent respectivement un afflux et un écoulement, et où les afflux des premières chambres partielles (122a, 123a) présentent une première conduite d'afflux commune (102a), et les afflux des deuxièmes chambres partielles (122b, 123b) présentent une deuxième conduite d'afflux commune (103a), et avec une installation d'évaluation (27),
**caractérisé**
**en ce que** l'installation d'évaluation (27) est configurée de telle sorte qu'en cours d'utilisation, elle détermine d'abord les temps de remplissage des premières chambres partielles (122a, 123a) et compare ensuite les temps de remplissage des premières chambres partielles (122a, 123a) des première et deuxième chambres d'équilibrage (122 respectivement 123) entre eux pour déterminer une différence des temps de remplissage des premières chambres partielles comme signe d'une fuite dans la paroi de séparation mobile.

2. Dispositif d'équilibrage selon la revendication 1, **caractérisé en ce que** l'installation d'évaluation (27) détermine de plus d'abord les temps de remplissage des deuxièmes chambres partielles (122b, 123b) et compare ensuite les temps de remplissage des deuxièmes chambres partielles (122b, 123b) des première et deuxième chambres d'équilibrage (122 respectivement 123) entre eux.

3. Dispositif d'équilibrage selon la revendication 1 ou 2, **caractérisé en ce que** l'installation d'évaluation, lors d'un dépassement d'une différence de temps prédéfinie (ΔT) émet un signal de détection de fuite.

4. Dispositif d'équilibrage selon la revendication 3, **caractérisé en ce qu'**il présente un générateur de signaux optique et/ou acoustique (28).

5. Dispositif d'équilibrage selon la revendication 3, **caractérisé en ce que** l'installation d'évaluation émet le signal de détection de fuite seulement lorsque le dépassement de la différence de temps prédéfinie (ΔT) selon des critères prédéfinis se produit à plusieurs reprises.
